# EUROPÄISCHE PATENTSCHRIFT

(11) **EP 0 609 709 B1**
(45) Veröffentlichungstag und Bekanntmachung des Hinweises auf die Patenterteilung: **20.03.2002**
(21) Anmeldenummer: 94100764.3
(22) Anmeldetag: 20.01.1994
(51) Int. Cl.: G01N 33/44, G01N 33/00

(54) **Verfahren zur Bestimmung von Kontaminaten in Behältern**
Method for determining contaminants in containers
Méthode pour la détermination de contaminants dans des récipients

(30) Priorität: 30.01.1993 DE 4302657
(43) Veröffentlichungstag der Anmeldung: 10.08.1994
(73) Patentinhaber: KHS Maschinen- und Anlagenbau Aktiengesellschaft, 44143 Dortmund (DE)
(72) Erfinder: Heidrich, Hermann, Karl Dr., D-45355 Essen (DE); Paroth, Berthold, D-44289 Dortmund (DE); Peters, Marten, D-24939 Flensburg (DE); Strauchmann, Rüdiger, D-24943 Flensburg (DE)

(56) Entgegenhaltungen:
- EP-A- 0 306 307
- WO-A-92/10751
- WO-A-92/10752

## Beschreibung

Die Erfindung bezieht sich auf ein Verfahren zur Bestimmung von Kontaminaten in Behältern, wie Mehrwegflaschen, bei welchem die Behälter auf ihrem Transportweg zu einer Meßstation transportiert und ein im Behälter befindlicher Luftanteil entnommen und einer Analysestation, vorzugsweise einem Massenspektrometer zugeführt und bei Feststellung bestimmter Kontaminate ausgeschieden werden.

Es ist bekannt, zur Bestimmung von bestimmten Restinhaltsstoffen oder einer Kontamination von wiederverwendbaren Behältern wie Kunststoffflaschen und dgl. deren Luftvolumen zu überprüfen. Zu diesem Zwecke sind Inspektionsverfahren und -maschinen bekannt, die einen Teil des Luftvolumens einer zu überprüfenden Flasche beispielsweise durch in den Flaschenhohlraum einführbare Sonden ansaugen und einem Analysegerät, z. B. einem Massenspektrometer, zuführen. Mit diesem Verfahren können bestimmte Inhaltsstoffe oder deren Reste ermittelt und die entsprechenden Flaschen aussortiert werden. Entsprechende Vefahren sind aus der DE 40 38 993 A1 und der DE 40 38 994 A1 bekannt.
Insbesondere bei der Verwendung eines Massenspektrometers ist eine Meßzeit für derartige Überprüfungen von 120 - 250 msec erforderlich. Bei der erforderlichen kurzen Überprüfungszeit, insbesondere von Getränkeflaschen, erwachsen hierbei Probleme. Durch Anlagerungen in den Zuleitungen zu den Meßstellen treten vorwiegend bei hoher Kontaminatbelastung Memorieeffekte auf, wodurch es praktisch unmöglich wird, die im Transportfluß nächstfolgende Flasche eindeutig zu bewerten.

Aus der WO-A-9 210 751 oder WO-A-9 210 752 ist bekannt, Test gas in einer Station mit nur einer Meßeinrichtung sequentiell zu messen. Der jeweilige Sensor, dessen Signale einen bestimmten Schwellenwert überschreiten, wird für mehrere Meßzyklen außer Betrieb gesetzt.

Der Erfindung liegt nun die Aufgabe zugrunde, hier Abhilfe und ein Verfahren zu schaffen, mit welchem auch bei hoher Durchsatzleistung eine einwandfreie Überprüfung der Behälter sichergestellt ist.

Diese Aufgabe wird gemäß der Erfindung bei einem Verfahren der eingangs genannten Art dadurch gelöst, daß der Analysestation vorbestimmt ein Vorsensor vorgeschaltet ist, mit welchem bestimmte Kontaminate und die nachfolgende Analyse memoriefrei durchführbar ist.

Weitere Merkmale der Erfindung sind in den Unteransprüchen angegeben.

Mit diesem Verfahren ist der Vorteil gegeben, daß durch den Vorsensor eindeutige Kontaminate sowie auch Stoffe erfaßt werden, die vom Massenspektrometer infolge von Interferenzen oder anderen Randbedingungen nicht bestimmt werden können. Hierunter fallen beispielsweise Urin, Essig, frisches Motorenöl und dgl. Überdies entfallen bei Eingliederung eines Vorsensors die sonst üblichen Memorieeffekte im Bereich der Zuleitungen zum eingesetzten Massenspektrometer.

Es können verschiedenartige Vorsensoren eingesetzt werden. Auch ist es denkbar, unterschiedliche Vorsensoren miteinander zu verknüpfen, die dann als Detektorkombination die Aufgabe einer entsprechenden Vorschalteinheit übernehmen.

## Patentansprüche

1. Verfahren zur Bestimmung von Kontaminaten in Behältern, wie Mehrwegflaschen, bei welchem die Behälter auf ihrem Transportweg zu einer Meßstation transportiert und ein im Behälter befindlicher Luftanteil entnommen und einer Analysestation, vorvorzugsweise einem Massenspektrometer zugeführt und bei Feststellung bestimmter Kontaminate ausgeschieden werden, ***dadurch gekennzeichnet,* daß** der Analysestation ein Vorsensor vorgeschaltet ist, mit welchem bestimmte Kontaminate vorbestimmt werden und die nachfolgende Analyse memoriefrei durchführbar ist.

2. Verfahren nach Anspruch 1, ***dadurch gekennzeichnet,* daß** als Vorsensor ein cross-flow modulierter Photoionisationsdetektor vorgeschaltet ist.

3. Verfahren nach Anspruch 1, ***dadurch gekennzeichnet,* daß** als Vorsensor ein cross-flow modulierter Flammenionisationsdetektor vorgeschaltet ist.

4. Verfahren nach Anspruch 1, ***dadurch gekennzeichnet,* daß** als Vorsensor ein cross-flow modulierter Elektroneneinfang-Coronadetektor vorgeschaltet ist.

5. Verfahren nach Anspruch 1, **dadurch gekennzeichnet, daß** als Vorsensor ein cross-flow modulierter Halbleitersensor vorgeschaltet ist.

6. Verfahren nach Anspruch 1, **dadurch gekennzeichnet, daß** ein Chemilumineszenzdetektor zur Bestimmung von Urin der Analysestation und/oder dem Vorsensor vorgeschaltet ist.

7. Verfahren nach Anspruch 1, **dadurch gekennzeichnet, daß** mehrere Vorsensoren nach den Ansprüchen 2 - 6 als Detektorkombination vorgeschaltet sind.

## Claims

1. Method of determining contaminants in vessels, such as multipurpose bottles, wherein the vessels are transported along their path of conveyance to a measuring station, and a proportion of air, present in the vessel, is removed and supplied to an analysing station, preferably a mass spectrometer, and removed therefrom when specific contaminants are detected, **characterised in that** a preliminary sensor is connected upstream of the analysing station, specific contaminants being predetermined by said sensor, and the subsequent analysis being accomplishable in a memory-free manner.

2. Method according to claim 1, **characterised in that** a crossflow-modulated photo ionisation detector is connected upstream as the preliminary sensor.

3. Method according to claim 1, **characterised in that** a crossflow-modulated flame ionisation detector is connected upstream as the preliminary sensor.

4. Method according to claim 1, **characterised in that** a crossflow-modulated electron-capture corona detector is connected upstream as the preliminary sensor.

5. Method according to claim 1, **characterised in that** a crossflow-modulated semiconductor sensor is connected upstream as the preliminary sensor.

6. Method according to claim 1, **characterised in that** a chemiluminescence detector for determining urine is connected upstream of the analysing station and/or of the preliminary sensor.

7. Method according to claim 1, **characterised in that** a plurality of preliminary sensors according to claims 2 - 6 are connected upstream as a combination of detectors.

## Revendications

1. Procédé déterminant la présence de contaminants dans des récipients, tels que des bouteilles consignées, dans lequel les récipients sont transportés sur leur parcours vers un poste de mesure et une fraction d'air se trouvant dans le récipient est prélevée et acheminée vers un poste d'analyse, de préférence un spectromètre de masse, et les récipients sont éliminés si l'on constate la présence de certains contaminants, **caractérisé en ce qu'**est monté, en amont du poste d'analyse, un détecteur préliminaire, au moyen duquel certains contaminants peuvent être préalablement déterminés, ce qui permet d'effectuer l'analyse suivante sans effet de mémoire.

2. Procédé selon la revendication 1, **caractérisé en ce qu'**un détecteur à photo-ionisation modulé par courant transversal est monté en amont pour servir de détecteur préliminaire.

3. Procédé selon la revendication 1, **caractérisé en ce qu'**un détecteur par ionisation de flammes modulé par courant transversal est monté en amont pour servir de détecteur préliminaire.

4. Procédé selon la revendication 1, **caractérisé en ce qu'**un détecteur à effet Corona par capture d'électrons est monté en amont pour servir de détecteur préliminaire.

5. Procédé selon la revendication 1, **caractérisé en ce qu'**un détecteur à semi-conducteur modulé par courant transversal est monté en amont pour servir de détecteur préliminaire.

6. Procédé selon la revendication 1, **caractérisé en ce qu'**un détecteur à chimiluminescence, servant à détecter la présence d'urine, est monté en amont du poste d'analyse et/ou du détecteur préliminaire.

7. Procédé selon la revendication 1, **caractérisé en ce que** plusieurs détecteurs préliminaires selon les revendications 2 à 6 sont montés en amont, pour former un ensemble de détecteurs.
